# EUROPEAN PATENT APPLICATION

(11) **EP 4 566 515 A1**
(43) Date of publication of application: **11.06.2025**
(21) Application number: 24020341.4
(22) Date of filing: 05.12.2024
(51) Int. Cl.: A61B 5/00, A61B 5/11, G01N 29/00, A61B 8/00

(54) **A SYSTEM AND METHOD FOR CREATING AND MAPPING VIBRATIONS FOR MICRO-VIBRATION ASSESSMENT**

(30) Priority: 06.12.2023 IN 202341083037
(71) Applicant: Turtle Shell Technologies Private Limited, Bengaluru, Karnataka 560038 (IN)
(72) Inventor: Kadambi, Pooja, 560038 Bengaluru, Karnataka (IN); Mohan, Umesh, 560038 Bengaluru, Karnataka (IN)
(74) Representative: Nirwan, Prajwal

(57) **Abstract**

Disclosed is a system and method for creating and mapping vibrations for micro-vibration assessment. The system comprises one or more vibration creators (102) configured to generate one or more vibration signals comprising at least one of predetermined patterns and intensities in a medium. The one or more sensors (104) configured to detect and capture one or more vibration signals propagated in the medium. Furthermore, a vibration mapping unit (106) configured to analyse the captured vibration signals to generate a vibration map. Thereafter, an artificial intelligence processing unit (108) configured to isolate and identify one or more of individual signals, signal patterns, and signatures of the captured vibrations signals by using the vibration map communicated by the vibration mapping unit (106), for providing an analysis of the micro-vibrations to a user device (110).

## Description

### TECHNICAL FIELD

The present disclosure generally relates to a vibration signal management and analysis. More specifically, the present disclosure relates to a system and method for creating and mapping vibrations for micro-vibration assessment.

### BACKGROUND

Micro-vibration sensing is crucial because it helps monitor heart health without invasive procedures. Ballistocardiography (BCG) measures body movements caused by the heartbeat, aiding in early detection of cardiac issues. Micro-vibration sensing tracks tiny vibrations in the body, offers insights into muscle activity and aids in diagnosing various health conditions.

In existing Ballistocardiography (BCG) and micro vibration sensing technologies, the accuracy and reliability has been limited, thus making it challenging to detect subtle changes in physiological parameters. Additionally, the overcorrection of gain and filtering has often led to a loss of resolution.

Currently, existing systems do not succeed in providing accurate and real-time feedback required for monitoring physiological parameters in dynamic situations such as patient movement or changing environmental conditions.

Other existing systems have tried to address this problem, but their scope was limited to using traditional piezoelectric-based Ballistocardiography and micro vibration sensing technologies, which often do not provide a complete information of physiological parameters, including pressure points and weight distribution.

Furthermore, when dealing with complex waveforms that combine signals and various types of noise (stationary and non-stationary), traditional algorithms often prioritize signal extraction while disregarding the magnitude and nature of noise within reasonable limits. Conventional algorithms can recognize signals as signals but often struggle to differentiate noise from signal, especially in real-world scenarios with suboptimal signal-to-noise ratios.

Thus, in light of the above discussion, it is evident that there is a need for a system and method that overcomes the limitations of existing technologies, offering reliability and addressing the challenges associated with accuracy, real-time feedback, and comprehensive physiological parameter monitoring.

### SUMMARY

The aim (i.e., principal object) of this invention is to provide a system and method to accurately detect and analyze micro-vibrations in a medium. The aim of the present disclosure is achieved by the system and method which accurately detect and analyze micro-vibrations in a medium.

A further object of the invention is to provide a system and method for integrating one or more sensors, such as piezoelectric and force sensors, to gather additional information about pressure points, weight distribution, and physiological parameters within the medium.

Another object of the invention is to incorporate artificial intelligence (AI) into the system to improve the accuracy and reliability of micro-vibration analysis.

Another object of the invention is to offer real-time feedback and monitoring capabilities.

Another object of the invention is to provide a comprehensive assessment of physiological parameters, including pressure points and weight distribution, enhancing the understanding of the medium's characteristics.

Another object of the invention is to enable gain correction to adjust sensor sensitivity for accurate micro-vibration detection.

Another object of the invention is to manage the activation, timing, and intensity of vibration creators for optimized data collection.

Another object of the invention is to process and transmit vibration signals efficiently for analysis and monitoring purposes.

In a first aspect, the present disclosure provides a system for creating and mapping vibrations for micro-vibration assessment, the system comprising:
one or more vibration creators configured to generate one or more vibration signals comprising at least one of predetermined patterns and intensities in a medium;
one or more sensors configured to detect and capture one or more vibration signals propagated in the medium;
a vibration mapping unit configured to analyse the captured vibration signals to generate and communicate a vibration map; and
an artificial intelligence processing unit configured to isolate and identify one or more of individual signals, signal patterns, and signatures of the captured vibrations signals by using the vibration map communicated by the vibration mapping unit, for providing an analysis of the micro-vibrations to a user device.

In an embodiment, the one or more vibration creators comprise at least one of piezoelectric transducers, electromagnetic actuators, acoustic transducers, vibration motors, pneumatic actuators, mechanical shakers, and ultrasonic transducers, and wherein the one or more sensors comprise at least one of piezoelectric sensors and force sensors for enabling detection of at least one of pressure, weight distribution, and one or more physiological parameters within the medium.

In an embodiment, the medium comprises at least one of a human body, a mattress, furniture, a civil infrastructure, and a material, wherein the medium is an environment or substance used to assess and analyze micro-vibrations.

In an embodiment, the one or more sensors comprise at least one of piezoelectric sensors and force sensors for enabling detection of at least one of pressure, weight distribution, and one or more physiological parameters within the medium.

In an embodiment, the force sensors are configured to sense one or more forces applied on the medium from external sources and communicate the detected vibrations or forces to the vibration mapping unit, wherein the vibration mapping unit is configured to identify one or more material properties to optimize signal conditioning and measuring resulting abnormalities in the medium.

In an embodiment, the vibration map generated by the vibration mapping unit comprises a visual representation of the vibration signals captured by the one or more sensors.

In an embodiment, the vibration mapping unit comprises:
an artificial intelligence mapping module configured to analyze the vibration map and identify patterns to enhance the accuracy and reliability of the system;
a noise correction module configured to identify and filter unwanted environmental vibrations or noise that interfere with accurate detection of micro-vibrations;
a gain correction module configured to adjust the sensitivity of the one or more sensors to ensure accurate detection of clean vibration signals, optimizing the system performance by accounting for variations in signal strength;
an environment assessment module configured to assess and analyse the environmental conditions of the medium, and factors comprising temperature, humidity, and external vibrations, to provide context for vibration data;
a medium assessment module configured to assess and analyse the at least one of pressure, weight distribution, and one or more physiological parameters of the medium;
a controller configured to manage the activation, timing, and intensity of the one or more vibration creators, ensuring control over the vibration generation process;
a power unit configured to supply power to one or more components of the system, ensuring continuous and stable operation;
a conditioning unit configured to prepare the detected and captured one or more sensors data for analysis, wherein the analysis comprises amplification, filtering, and other pre-processing of captured one or more sensors data;
a transmitter configured to transmit the one or more sensors data to a remote receiver, enabling remote data analysis and real-time monitoring of the medium;
a converter configured to transform input signals into a vectorized format in a windowed fashion, facilitating the capture of time, frequency, and domain-specific signal features, thereby enabling advanced signal processing and analysis; and
the receiver configured to collect and receive the one or more sensors data transmitted by the transmitter, which is further processed or displayed for user monitoring or analysis.

In an embodiment, the artificial intelligence processing unit comprises at least one generative model comprising at least one of Generative Adversarial Networks (GANs) and Large Language Models (LLMs), to derive the one or more individual signals of the one or more captured vibration signals.

In an embodiment, the artificial intelligence processing unit comprises:
a training module configured to generate at least one training set incorporating clean and standardized signals, and apply one or more of sources, levels, and interactions of the one or more individual signals to produce the clean vibration signals; and
a Fast Fourier Transform (FFT) mapping module configured to compute at least one Fast Fourier Transform (FFT) of the one or more captured vibration signals.

In an embodiment, the one or more individual signals comprise one or more of a clean vibration signal, a medium noise, an environmental noise, an object noise, a stationary noise and a non-stationary noise.

In an embodiment, the user device is configured to monitor the vibrations in real time and analyze the one or more individual signals provided by the artificial intelligence processing unit and facilitating real time feedback to a user.

In a second aspect, the present disclosure provides a method for creating and mapping vibrations for micro-vibration assessment, the method comprising:
generating, by one or more vibration creators, one or more vibration signals comprising at least one of predetermined patterns and intensities in a medium;
detecting and capturing, by one or more sensors, one or more vibration signals propagated in the medium;
analyzing, by a vibration mapping unit, the captured vibration signals to generate and communicate a vibration map; and
isolating and identifying, by an artificial intelligence processing unit, one or more of individual signals, signal patterns, and signatures of the captured vibrations signals by using the vibration map communicated by the vibration mapping unit, for providing an analysis of the micro-vibrations to a user device.

In an embodiment, the one or more sensors comprise at least one of piezoelectric sensors and force sensors for enabling detection of at least one of pressure, weight distribution, and one or more physiological parameters within the medium.

In an embodiment, the method further comprises configuring the force sensors for sensing one or more forces applied on the medium from external sources, and communicating the detected vibrations or forces to the vibration mapping unit, wherein the vibration mapping unit is configured for identifying one or more material properties to optimize signal conditioning and measuring resulting abnormalities in the medium.

In an embodiment, the method further comprises generating the vibration map comprising a visual representation of the vibration signals captured by the one or more sensors.

In an embodiment, the method further comprises configuring the vibration mapping unit by:
analyzing, by an artificial intelligence mapping module, the vibration map and identifying patterns to enhance the accuracy and reliability of a system;
identifying and filtering, by a noise correction module, unwanted environmental vibrations or noise that interfere with accurate detection of micro-vibrations;
adjusting, by a gain correction module, the sensitivity of the one or more sensors to ensure accurate detection of clean vibration signals, optimizing the system performance by accounting for variations in signal strength;
assessing and analyzing, by an environment assessment module, the environmental conditions of the medium, and factors comprising temperature, humidity, and external vibrations, to provide context for vibration data;
assessing and analyzing, by a medium assessment module, the at least one of pressure, weight distribution, and one or more physiological parameters of the medium;
managing, by a controller, the activation, timing, and intensity of the one or more vibration creators, ensuring control over the vibration generation process;
   supplying, by a power unit, power to one or more components of the system, ensuring continuous and stable operation;
preparing, by a conditioning unit, the detected and captured one or more sensors data for analysis, wherein the analysis comprises amplification, filtering, and other pre-processing of captured one or more sensors data;
transmitting, by a transmitter, the one or more sensors data to a remote receiver, enabling remote data analysis and real-time monitoring of the medium;
transforming, by a converter, input signals into a vectorized format in a windowed fashion, facilitating the capture of time, frequency, and domain-specific signal features, thereby enabling advanced signal processing and analysis; and
collecting and receiving, by the receiver, the one or more sensors data transmitted by the transmitter, which is further processed or displayed for user monitoring or analysis.

In an embodiment, the method further comprises configuring the artificial intelligence processing unit by:
generating, by a training module, at least one training set incorporating clean and standardized signals, and applying one or more of sources, levels, and interactions of the one or more individual signals to produce the clean vibration signals; and
computing, by Fast Fourier Transform (FFT) mapping module, at least one Fast Fourier Transform (FFT) of the one or more captured vibration signals.

In an embodiment, the method further comprises monitoring, by the user device, the vibrations and analyzing the one or more individual signals, in real-time, provided by the artificial intelligence processing unit and facilitating real-time feedback to a user.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

This invention is illustrated in the accompanying drawings, throughout which, like reference letters indicate corresponding parts in the various figures.

The embodiments herein will be better understood from the following description with reference to the drawings, in which:
Figure 1 depicts a system for creating and mapping vibrations for micro-vibration assessment, in accordance with an embodiment of the present disclosure;
Figure 2a illustrates a block diagram depicting components for creating and mapping vibrations for micro-vibration assessment, in accordance with an embodiment of the present disclosure;
Figure 2b illustrates a block diagram depicting components of vibration mapping unit, in accordance with an embodiment of the present disclosure;
Figure 3a depicts an artificial intelligence processing unit utilized for micro-vibration assessment, in accordance with an embodiment of the present disclosure;
Figure 3b depicts the artificial intelligence processing unit utilized for micro-vibration assessment, in accordance with an embodiment of the present disclosure;
Figure 4 illustrates a method for creating and mapping vibrations for micro-vibration assessment, in accordance with an embodiment of the present disclosure;
Figure 5a illustrates a method for creating and mapping vibrations for micro-vibration assessment, in accordance with an embodiment of the present disclosure; and
Figure 5b illustrates a method for creating and mapping vibrations for micro-vibration assessment, in accordance with an embodiment of the present disclosure.

### DETAILED DESCRIPTION

The embodiments herein and the various features and advantageous details thereof are explained more fully with reference to the non-limiting embodiments that are illustrated in the accompanying drawings and/or detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

The present invention discloses a system and method for creating and mapping vibrations for micro-vibration assessment.

The system comprises one or more vibration creators configured to generate one or more vibration signals comprising at least one of predetermined patterns and intensities in a medium.

Furthermore, the system comprises one or more sensors configured to detect and capture one or more vibration signals propagated in the medium.

Furthermore, the system comprises a vibration mapping unit configured to analyse the captured vibration signals to generate a vibration map.

Thereafter, the system comprises an artificial intelligence processing unit configured to isolate and identify one or more of individual signals, signal patterns, and signatures of the captured vibrations signals by using the vibration map communicated by the vibration mapping unit, for providing an analysis of the micro-vibrations to a user device.

Vibration mapping is crucial for micro-vibration sensing and environment assessment because it helps identify and locate tiny vibrations that might go unnoticed but can have significant impacts. This vibration mapping helps to understand and monitor the health and stability of a patient, delicate structures, such as buildings or electronic devices, and aids in early detection of potential issues, ensuring safety and efficiency.

The present invention discloses a system and a method for vibration mapping and micro-vibration assessment. The system utilizes a mesh of specialized vibration creators strategically placed within a predefined proximity to a medium, which is then monitored by advanced piezoelectric and force sensors. The resulting vibration data is processed through digital signal processing and artificial intelligence units, facilitating real-time analysis, noise cancellation, and gain correction.

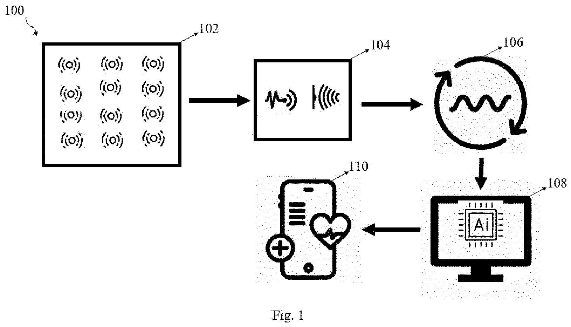

Figure 1 depicts a system 100 for creating and mapping vibrations for micro-vibration assessment, in accordance with an embodiment of the present disclosure. The system comprises one or more vibration creators 102, one or more sensors 104, a vibration mapping unit 106, an artificial intelligence processing unit 108, a user device 110.

In an embodiment, the one or more vibration creators 102 comprise at least one of piezoelectric transducers, electromagnetic actuators, acoustic transducers, vibration motors, pneumatic actuators, mechanical shakers, and ultrasonic transducers.

In an embodiment, the one or more vibration creators 102 may be configured to generate controlled and precise vibrations in a medium. The one or more vibration creators 102 are placed in strategic locations of the medium to create a mesh of vibrations for micro-vibration assessment taking place within the medium.

In an embodiment, the one or more vibration creators 102 can be positioned around a patient's support component such as bed or chair to monitor and sense the micro-vibrations, comfort, and vital signs of the patient.

In an embodiment, the medium may be an environment or substance in which the system 100 is deployed to assess and analyze micro-vibrations. The medium may be at least one of a human body, a mattress, furniture, a civil infrastructure, a material and the like, wherein the medium is an environment or substance used to assess and analyze micro-vibrations.

In an embodiment, the one or more sensors 104 may comprise at least one of piezoelectric sensors and force sensors for enabling detection of at least one of pressure, weight distribution, and one or more physiological parameters within the medium.

The piezoelectric sensors may be configured to detect micro-vibrations, and the force sensors may be configured to detect pressure, weight distribution, and physiological parameters within the medium.

Optionally, the force sensors are also configured to sense one or more forces applied on the medium from external sources and communicate the detected vibrations or forces to the vibration mapping unit 106, wherein the vibration mapping unit 106 is configured to identify one or more material properties to optimize the signal conditioning and measuring the resulting abnormalities in the medium (i.e., deformation upon application of said force and pressure).

In an embodiment, the one or more vibration sensors 104 may be configured to sense known forces or pressures applied to the medium, ensuring accurate measurements and calibration of the system.

In an embodiment, the noise in the medium refers to any vibrations or disturbances in the medium itself (the environment or substance where the vibration mapping system is deployed) that are not part of the clean vibration signal. These vibrations could arise from various sources within the medium, such as natural movements, fluid flow, or structural vibrations.

In an embodiment, the environment noise encompasses external vibrations and disturbances originated from the surroundings of the medium. The environment noise may come from sources outside the medium, such as nearby machinery, vehicles, or environmental factors like wind or seismic activity.

In an embodiment, the object noise relates to vibrations caused by specific objects or components within the medium that are not part of the clean vibration signal. For example, if the system is used to assess vibrations in a mechanical system, the vibrations generated by individual components of that system would be considered object noise.

In an embodiment, the stationary noise represents constant or unchanging disturbances in the vibration signal over time. This type of noise may include low-frequency vibrations or interference that persist throughout the duration of the signal.

In an embodiment, the non-stationary noise refers to disturbances in the vibration signal that vary or change over time. These variations may be sudden or gradual and can include transient spikes or fluctuations.

In an embodiment, the vibration mapping unit 106 may be a digital signal processing (DSP) unit or any processing unit. The vibration mapping unit 106 is configured to analyze the vibration signals detected and communicated by the one or more vibration sensors, in order to detect one or more clean vibration signals. Herein, the clean or original vibration signals are also referred to as individual signals. The one or more individual signals comprise one or more of a clean vibration signal, a medium noise, an environmental noise, an object noise, a stationary noise and a non-stationary noise.

In an embodiment, the clean vibration signal is generated by the one or more vibration creators 102 in the absence of any unwanted interference or noise. The clean vibration signal represents the ideal or target signal that the system aims to capture.

Upon analyzing the vibration signals, the vibration mapping unit 106 generates at least one vibration map that provides a detailed assessment of changes in the medium due to vibrations generated by the one or more vibration creators 102. Optionally, the vibration maps generated by the vibration mapping unit 106 comprises a visual representation of the vibration signals captured by the one or more sensors 104. Additionally, the vibration mapping unit 106 may comprise adaptive noise cancellation capabilities to filter out unwanted noise.

In an embodiment, the artificial intelligence processing unit 108 is configured to analyze the vibration map and identifying patterns or anomalies using machine learning algorithms, such as Generative Adversarial Networks (GANs) and Large Language Models (LLMs).

In an embodiment, the user device 110 may comprise at least one of wearable devices, mobile phones, Personal Digital Assistant (PDA), smartphones, smart band, smart watch, laptop, computer and the like with a user-friendly interface. The user device 110 may be configured for real-time monitoring of vibrations and analyzing the clean signals provided by the artificial intelligence processing unit 108. The system 100 may comprise as many user devices 110 as required by the user.

In an embodiment, the user device 110 may also comprise an interface module that further comprises one or more hardware, software and firmware components for receiving, sharing and displaying data or signal from other devices including the user devices 110.

In an embodiment, the communication module of the user device 110 may include wired and wireless communication, including but not limited to, GPS, GSM, LAN, Wi-fi compatibility, Bluetooth low energy as well as NFC. The wireless communication may further comprise one or more of Bluetooth (registered trademark), ZigBee (registered trademark), a short-range wireless communication such as UWB, a medium-range wireless communication such as WiFi (registered trademark) or a long-range wireless communication such as 3G/4G or WiMAX (registered trademark), according to the usage environment.

In an embodiment, the memory module of the user device 110 comprises one or more volatile and non-volatile memory components which are capable of storing data and instructions to be executed.

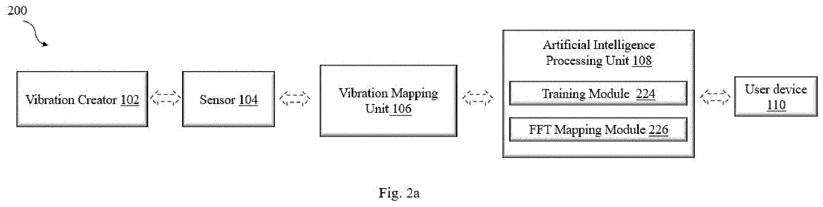

Figure 2a illustrates a block diagram depicting components for creating and mapping vibrations for micro-vibration assessment, in accordance with an embodiment of the present disclosure.

Firstly, the one or more vibration creators 102 comprise piezoelectric transducers that are placed in strategic locations in the proximity to the medium, such as a mattress, to generate controlled and precise vibrations for micro-vibration assessment.

Furthermore, the one or more sensors 104 comprise piezoelectric sensors and force sensors. These sensors are distributed in strategic locations on the medium or can be placed in proximity of the medium to detect and capture the vibrations generated by the one or more vibration creators 102. The piezoelectric sensors and force sensors may be used for enabling detection of at least one of pressure, weight distribution, and one or more physiological parameters within the medium. They play a critical role in calibrating the system for precise measurements by applying known forces or pressures to establish baseline readings. The one or more sensors 104 may also sense material properties, such as elasticity and stiffness, by subjecting materials to known forces or pressures and measuring resulting deformations, thereby optimizing signal conditioning.

Overall, the combination of the piezoelectric sensors and the force sensors can enhance the vibration mapping system by providing additional information about pressure, weight distribution, physiological parameters, and material properties.

Furthermore, the vibration mapping unit 106 is configured for analyzing the vibration signals captured by the one or more sensors 104. It generates a vibration map that provides a detailed assessment of changes in the medium due to vibrations.

Furthermore, the artificial intelligence processing unit 108, as part of the system 100, comprises a training module 224 and a Fast Fourier Transform (FFT) mapping module 226. The training module 224 is configured to train neural networks to analyze the vibration map and identify patterns or anomalies. The FFT mapping module 226 is configured for computing at least one Fast Fourier transform (FFT) of the composite signal, allowing for more precise signal extraction. The training module 224 is configured to generate at least one training set incorporating clean and standardized signals, and apply one or more of sources, levels, and interactions of the one or more individual signals to produce the clean vibration signals, and the FFT mapping module 226 is configured to compute at least one Fast Fourier Transform (FFT) of the one or more captured vibration signals.

In an embodiment, the artificial intelligence processing unit 108 is configured to analyze the vibration map and identify patterns or anomalies using machine learning algorithms, such as Generative Adversarial Networks (GANs) and Large Language Models (LLMs).

In an embodiment, the FFT mapping module 226 is configured to process the one or more vibration signals by computing the Fast Fourier Transform (FFT) to extract frequency domain information, enhancing the precision of the analysis.

Thereafter, the user device 110, such as a computer or a mobile device, is configured to monitor the vibrations in real-time and analyze clean vibration signals provided by the processing unit. The user-friendly interface of the user device 110 facilitates real time feedback and insights for various applications, including healthcare, sports, and product design.

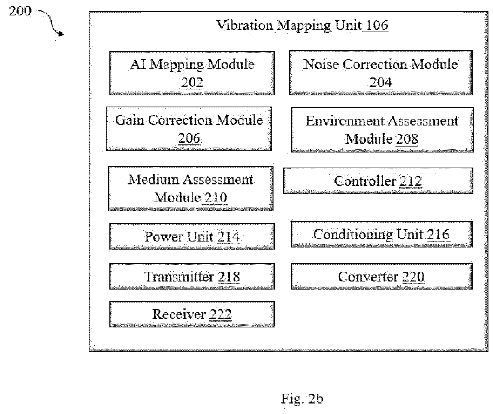

Figure 2b illustrates a block diagram depicting components of vibration mapping unit 106, in accordance with an embodiment of the present disclosure.

In an embodiment, the vibration mapping unit 106 comprises an artificial Intelligence (AI) mapping module 202, a noise correction module 204, a gain correction module 206, an environment assessment module 208, a medium assessment module 210, a controller 212, a power unit 214, a conditioning unit 216, a transmitter 218, a converter 220, and a receiver 222.

In an embodiment, the artificial Intelligence (AI) mapping module 202 is configured to analyze the vibration map and identify patterns or anomalies to enhance the accuracy and reliability of the system. The Artificial Intelligence (AI) mapping module 202 may comprise machine learning algorithms, including Generative Adversarial Networks (GANs) and Large Language Models (LLMs). These algorithms enhance the accuracy and reliability of the system.

In an embodiment, the noise correction module 204 is configured to filter out unwanted environmental vibrations or noise that may interfere with the accurate detection of micro-vibrations. The noise correction module 204 employs advanced signal processing techniques to improve signal quality.

In an embodiment, the gain correction module 206 is configured to adjust the sensitivity of the one or more sensors 104 to ensure they accurately detect clean vibration signals (i.e., the desired signals). The gain correction module 206 uses information from the vibration map to optimize the one or more sensors 104 sensitivity, improving data accuracy. The gain correction module 206 optimizes the system performance by accounting for variations in signal strength.

In an embodiment, the environment assessment module 208 is configured to assess and analyze the environmental conditions of the medium in which the system is deployed, the environment assessment module 208 monitors factors such as temperature, humidity, external vibrations and ambient noise levels to provide a comprehensive assessment. This provides context for vibration data.

In an embodiment, the medium assessment module 210 is configured to assess and analyze the characteristics of the medium in which the system is deployed, such as a mattress or human body. The medium assessment module 210 evaluates parameters like pressure points, weight distribution, and material properties for optimizing support and comfort. The medium assessment module 210 is configured to assess and analyse the at least one of pressure, weight distribution, and one or more physiological parameters of the medium

In an embodiment, the controller 212 is configured to manage the activation, timing, and intensity of the one or more vibration creators 102. The controller 212 ensures precise control over the generated vibrations in the vibration generation process, allowing for various assessment scenarios.

In an embodiment, the power unit 214 is configured to supply the necessary power to all components of the system, including the one or more vibration creators 102, the one or more sensors 104, the vibration mapping unit 106, and the artificial intelligence processing unit 108. It ensures uninterrupted (i.e., continuous) and stable operation.

In an embodiment, the conditioning unit 216 is configured to preprocess detected and captured one or more sensors data of the vibration signals for analysis. The conditioning unit 216 extracts valuable information from the captured data by the one or more sensors 104 to enhance the accuracy of subsequent processing. The analysis comprises amplification, filtering, and other pre-processing of captured one or more sensors data.

In an embodiment, the transmitter 218 is configured to transmit a processed one or more sensors data to a receiver 222. The transmitter 218 enables data transfer between different parts of the system. This enables remote data analysis and real-time monitoring of the medium.

In an embodiment, the converter 220 is configured to transform input signals into a vectorized format in a windowed fashion, facilitating the capture of time, frequency, and domain-specific signal features. This format enhances the precision of signal analysis within subsequent processing stages. In an embodiment, the input signal may be the one or more vibration signals detected and captured by the one or more sensors 104.

In an embodiment, the receiver 222 is configured to collect and receive transmitted one or more sensors data, which is further processed or displayed for user monitoring or analysis.

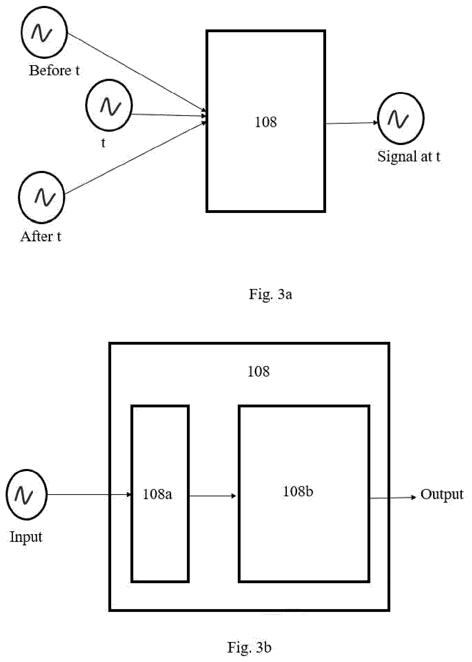

Figure 3a depicts an artificial intelligence processing unit 108 utilized for micro-vibration assessment, in accordance with an embodiment of the present disclosure.

The artificial intelligence processing unit 108 may comprise at least one of a neural network model, a machine learning model or an artificial intelligence model in the context of vibration signal analysis.

To achieve noise characterization, the training module 224 is created. This module comprises "clean" standard or normalized signals to which various sources, levels, and interactions of noise are applied. This process helps build patterns that capture different noises related to the environment, subjects, and medium in which the system operates.

The artificial intelligence processing unit 108 employs the self-supervised or unsupervised model with a complex function designed to generate a Fast Fourier Transform (FFT) of the composite signal and isolate individual signature components, including both signal and noise. As shown in figure 3a, the analysis of signals involves examining them at specific time points (t) as well as considering the information both before and after those time points to extract meaningful features and patterns.

At time t, the neural network examines the signal or data point at that specific moment. This analysis includes assessing the properties, characteristics, and features of the signal at that time instance. For instance, in the case of micro-vibration sensing, the signal at time t might represent a specific vibration reading or data point captured by the one or more sensors 104.

Before time t, the neural network also considers the signal data points that occurred before time t. This historical information provides valuable context and may reveal trends or patterns leading up to the current moment, i.e., time t. It helps the network understand how the signal has been evolving.

After time t, the neural network may also consider signal data points that occur after time t. This forward-looking analysis helps in predicting or anticipating how the signal might continue to evolve. It can be useful for tasks such as identifying trends or predicting future changes in the signal.

By considering past and future signal data points, the neural network gains a more comprehensive understanding of the signal's behavior. This context is crucial for accurately distinguishing between the desired vibration signal and various noise components. Analyzing data over time allows the neural network to recognize patterns and trends in the signal. This can help in identifying specific vibration patterns associated with micro-vibrations, which might otherwise be challenging to detect in isolation. Understanding the changes in the signal over time enables the system to better distinguish between the clean vibration signal and various types of noise. This aids in noise reduction and improves the precision of vibration analysis. Neural networks can adapt and learn from historical and future data to make real-time adjustments. This adaptability is useful for maintaining the system's accuracy in dynamic environments.

Figure 3b depicts artificial intelligence processing unit 108 utilized for micro-vibration assessment, in accordance with an embodiment of the present disclosure.

To enable more effective learning, the input signal undergoes conversion into a vector format using a windowed approach. This method captures time, frequency, and domain-specific signal features (e.g., peaks in an ECG signal) rather than working with a one-dimensional representation, the system operates in a higher-dimensional format. This enriched data is subsequently passed to neural networks for improved learning.

The sequence of vectors 108a facilitates the use of transformer architecture with attention mechanisms. This enables the neural network to automatically learn which surrounding areas of the waveform (context) to consider while processing a particular part of the waveform, enhancing the precision of the analysis.

The algorithms used by the artificial intelligence processing unit 108 also models noise using generative models like Generative Adversarial Networks (GANs), Stable Diffusion, or Large Language Models (LLMs). Optionally, the artificial intelligence processing unit comprises at least one generative model comprising at least one of Generative Adversarial Networks (GANs) and Large Language Models (LLMs), to derive the one or more individual signals of the one or more captured vibration signals. These models are designed to represent both noise and signal components of complex waveforms and have parameterized settings for generating these components with varying amplitudes, frequencies, and other domain-specific parameters (e.g., heart rate). The training module 224 is generated with randomized combinations of signal and noise components, including randomized parameters. This process results in a substantial training dataset of complex waveforms. A Deep Neural Network (DNN) 108b may also be employed to train the model further. This DNN 108 is tasked with decomposing a given complex waveform into its constituent noise and signal components, leveraging the vectorized input signal.

Due to the vectorized input signal, transformer networks with attention mechanisms are employed. These mechanisms enhance precision by allowing the network to focus on relevant areas of the waveform during processing.

The artificial intelligence processing unit 108 plays a critical role in enhancing the accuracy and reliability of micro-vibration signal analysis by effectively addressing noise, optimizing signal representation, and facilitating advanced learning mechanisms.

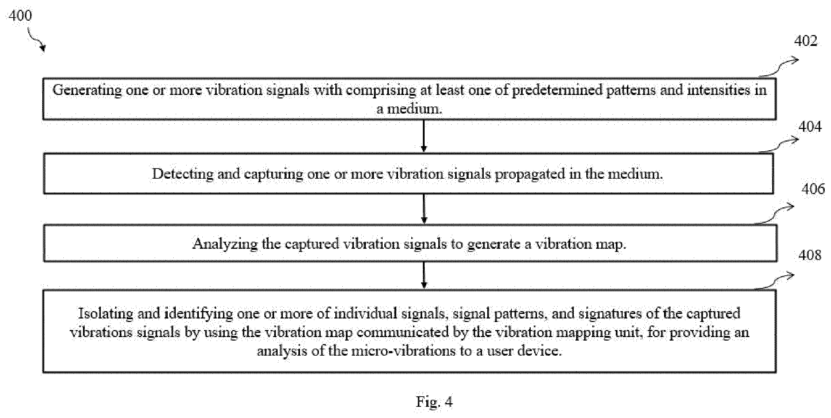

Figure 4 illustrates a method for creating and mapping vibrations for micro-vibration assessment, in accordance with an embodiment of the present disclosure. The method 400 begins with generating one or more vibration signals comprising at least one of predetermined patterns and intensities in a medium by one or more vibration creators 102, as depicted in step 402. Furthermore, the method 400 discloses detecting and capturing one or more vibration signals propagated in the medium by one or more sensors 104, as depicted in step 404. Additionally, the method 400 discloses analyzing the captured vibration signals to generate a vibration map by a vibration mapping unit 106, as depicted in step 406. Thereafter, the method 400 discloses isolating and identifying, by an artificial intelligence processing unit 108, one or more of individual signals, signal patterns, and signatures of the captured vibrations signals by using the vibration map communicated by the vibration mapping unit 106, for providing an analysis of the micro-vibrations to a user device 110, as depicted in step 408.

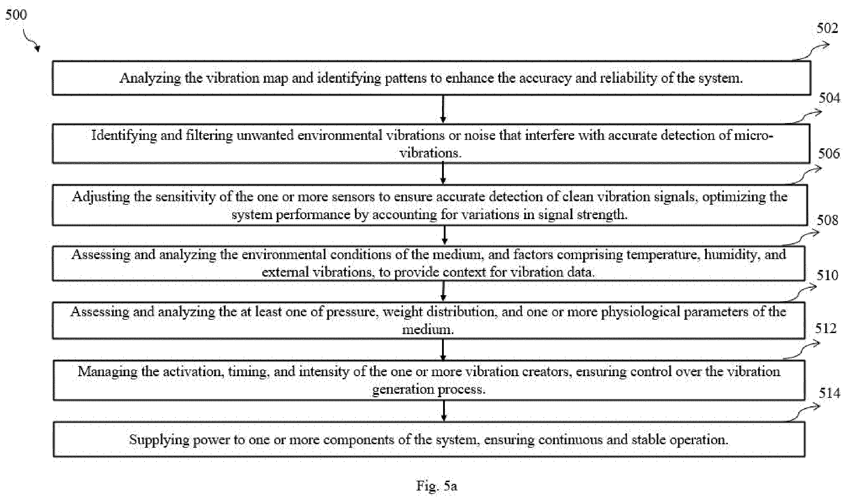

Figure 5a illustrates a method for creating and mapping vibrations for micro-vibration assessment, in accordance with an embodiment an embodiment of the present disclosure. The method 500 begins with analyzing, by an artificial intelligence mapping module 202, the vibration map and identifying patterns to enhance the accuracy and reliability of the system, as depicted in step 502. Subsequently, the method 500 discloses identifying and filtering, by a noise correction module 204, unwanted environmental vibrations or noise that interfere with accurate detection of micro-vibrations, as depicted at step 504. Furthermore, the method 500 discloses adjusting, by a gain correction module 206, the sensitivity of the one or more sensors to ensure accurate detection of clean vibration signals, optimizing the system performance by accounting for variations in signal strength, as depicted at step 506. Additionally, the method 500 discloses assessing and analyzing, by an environment assessment module 208, the environmental conditions of the medium, and factors comprising temperature, humidity, and external vibrations, to provide context for vibration data, as depicted at step 508. Subsequently, method 500 discloses assessing and analyzing, a medium assessment module 210, the at least one of pressure, weight distribution, and one or more physiological parameters of the medium, as depicted at step 510. Furthermore, method 500 discloses managing, by a controller 212, the activation, timing, and intensity of the one or more vibration creators 102, ensuring control over the vibration generation process, as depicted at step 512. Subsequently, the method 500 discloses supplying, by a power unit 214, power to one or more components of the system 100, ensuring continuous and stable operation, as depicted at step 514.

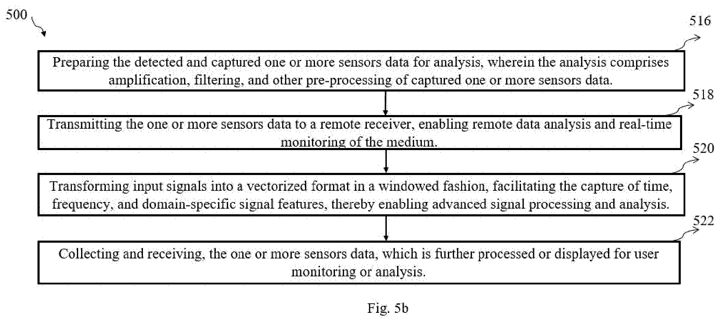

Figure 5b illustrates a method for creating and mapping vibrations for micro-vibration assessment, in accordance with an embodiment of the present disclosure. Subsequently, the method 500 discloses preparing, by a conditioning unit 216, the detected and captured one or more sensors data for analysis, wherein the analysis comprises amplification, filtering, and other pre-processing of captured one or more sensors data, as depicted at step 516. Subsequently, the method 500 discloses transmitting, by a transmitter 218, the one or more sensors data to a remote receiver 222, enabling remote data analysis and real-time monitoring of the medium, as depicted at step 518. Subsequently, the method 500 discloses transforming, by a converter 220, input signals into a vectorized format in a windowed fashion, facilitating the capture of time, frequency, and domain-specific signal features, thereby enabling advanced signal processing and analysis, as depicted at step 520. Thereafter, the method 500 discloses collecting and receiving, by the receiver 222, the one or more sensors data transmitted by the transmitter 218, which is further processed or displayed for user monitoring or analysis, as depicted at step 522.

The advantages of the current invention include providing enhanced accuracy and reliability in micro-vibration sensing, enabling the detection of subtle changes in physiological parameters.

An additional advantage is that the system provides an effective noise cancellation capability that improves signal quality, even in noisy environments.

An additional advantage is that the system provides real-time feedback for monitoring changes in physiological parameters, allowing timely adjustments.

An additional advantage is that the system provides comprehensive assessment of pressure points, weight distribution, and material properties, providing valuable insights into various applications.

An additional advantage is that the system provides the ability to create vibration maps for optimizing support and comfort in healthcare products and devices.

An additional advantage is that the system provides additional information on pressure, weight distribution, and material properties using the force sensors.

An additional advantage is that the system enables real-time monitoring and immediate feedback, which is crucial for healthcare applications where timely adjustments are required based on patient movement and comfort.

An additional advantage is that the system's noise cancellation capabilities ensure that the micro-vibrations of interest are accurately detected and analyzed, even in the presence of external noise.

An additional advantage is that the system's gain correction feature optimizes sensor sensitivity, preventing over-correction or loss of resolution in data.

An additional advantage is that the vibration mapping system can provide a complete picture of physiological parameters, including pressure points and weight distribution, contributing to better healthcare product design.

An additional advantage is that the system's integration of force sensors enhances its capabilities for assessing material properties, such as elasticity and stiffness, which is valuable for optimizing support structures. An additional advantage is that the system's AI algorithms and signal processing techniques significantly improve the accuracy and reliability of micro-vibration analysis.

Applications of the current invention include healthcare, sports, product design optimization, and environmental assessment.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the embodiments as described here.

## Claims

1. A system (100) for creating and mapping vibrations for micro-vibration assessment, the system (100) comprising:
one or more vibration creators (102) configured to generate one or more vibration signals comprising at least one of predetermined patterns and intensities in a medium;
one or more sensors (104) configured to detect and capture one or more vibration signals propagated in the medium;
a vibration mapping unit (106) configured to analyse the captured vibration signals to generate and communicate a vibration map; and
an artificial intelligence processing unit (108) configured to isolate and identify one or more of individual signals, signal patterns, and signatures of the captured vibrations signals by using the vibration map communicated by the vibration mapping unit (106), for providing an analysis of the micro-vibrations to a user device (110).

2. A system (100) according to claim 1, wherein the one or more vibration creators (102) comprise at least one of piezoelectric transducers, electromagnetic actuators, acoustic transducers, vibration motors, pneumatic actuators, mechanical shakers, and ultrasonic transducers, and wherein the one or more sensors (104) comprise at least one of piezoelectric sensors and force sensors for enabling detection of at least one of pressure, weight distribution, and one or more physiological parameters within the medium.

3. A system (100) according to any of the preceding claims, wherein the medium comprises at least one of a human body, a mattress, furniture, a civil infrastructure, and a material, wherein the medium is an environment or substance used to assess and analyze micro-vibrations.

4. A system (100) according to claim 2, wherein the force sensors are configured to sense one or more forces applied on the medium from external sources and communicate the detected vibrations or forces to the vibration mapping unit (106), wherein the vibration mapping unit (106) is configured to identify one or more material properties to optimize signal conditioning and measuring resulting abnormalities in the medium.

5. A system (100) according to any of the preceding claims, wherein the vibration map generated by the vibration mapping unit (106) comprises a visual representation of the vibration signals captured by the one or more sensors (104).

6. A system (100) according to any of the preceding claims, wherein the vibration mapping unit (106) comprises:
an artificial intelligence mapping module (202) configured to analyze the vibration map and identify patterns to enhance the accuracy and reliability of the system;
a noise correction module (204) configured to identify and filter unwanted environmental vibrations or noise that interfere with accurate detection of micro-vibrations;
a gain correction module (206) configured to adjust the sensitivity of the one or more sensors to ensure accurate detection of clean vibration signals, optimizing the system (100) performance by accounting for variations in signal strength;
an environment assessment module (208) configured to assess and analyse the environmental conditions of the medium, and factors comprising temperature, humidity, and external vibrations, to provide context for vibration data;
a medium assessment module (210) configured to assess and analyse the at least one of pressure, weight distribution, and one or more physiological parameters of the medium;
a controller (212) configured to manage the activation, timing, and intensity of the one or more vibration creators (102), ensuring control over the vibration generation process;
a power unit (214) configured to supply power to one or more components of the system (100), ensuring continuous and stable operation;
a conditioning unit (216) configured to prepare the detected and captured one or more sensors data for analysis, wherein the analysis comprises amplification, filtering, and other pre-processing of captured one or more sensors data;
a transmitter (218) configured to transmit the one or more sensors data to a remote receiver (224), enabling remote data analysis and real-time monitoring of the medium;
a converter (220) configured to transform input signals into a vectorized format in a windowed fashion, facilitating the capture of time, frequency, and domain-specific signal features, thereby enabling advanced signal processing and analysis; and
the receiver (222) configured to collect and receive the one or more sensors data transmitted by the transmitter (218), which is further processed or displayed for user monitoring or analysis.

7. A system (100) according to any of the preceding claims, wherein the artificial intelligence processing unit (108) comprises at least one generative model comprising at least one of Generative Adversarial Networks (GANs) and Large Language Models (LLMs), to derive the one or more individual signals of the one or more captured vibration signals.

8. A system (100) according to any of the preceding claims, wherein the artificial intelligence processing unit (108) comprises:
a training module (224) configured to generate at least one training set incorporating clean and standardized signals, and apply one or more of sources, levels, and interactions of the one or more individual signals to produce the clean vibration signals; and
a Fast Fourier Transform (FFT) mapping module (226) configured to compute at least one Fast Fourier Transform (FFT) of the one or more captured vibration signals.

9. A system (100) according to any of the preceding claims, wherein the user device (110) is configured to monitor the vibrations in real time and analyze the one or more individual signals provided by the artificial intelligence processing unit (108) and facilitating real time feedback to a user.

10. A method (400) for creating and mapping vibrations for micro-vibration assessment, the method (400) comprising:
generating, by one or more vibration creators (102), one or more vibration signals comprising at least one of predetermined patterns and intensities in a medium;
detecting and capturing, by one or more sensors (104), one or more vibration signals propagated in the medium;
analyzing, by a vibration mapping unit (106), the captured vibration signals to generate and communicate a vibration map; and
isolating and identifying, by an artificial intelligence processing unit (108), one or more of individual signals, signal patterns, and signatures of the captured vibrations signals by using the vibration map communicated by the vibration mapping unit (106), for providing an analysis of the micro-vibrations to a user device (110).

11. A method (400) according to claim 10, wherein the one or more sensors (104) comprise at least one of piezoelectric sensors and force sensors for enabling detection of at least one of pressure, weight distribution, and one or more physiological parameters within the medium.

12. A method (400) according to claim 11, further comprising configuring the force sensors for sensing one or more forces applied on the medium from external sources, and communicating the detected vibrations or forces to the vibration mapping unit (106), wherein the vibration mapping unit (106) is configured for identifying one or more material properties to optimize signal conditioning and measuring resulting abnormalities in the medium.

13. A method (400) according to any of claims 10-12, further comprising configuring the vibration mapping unit (106) by:
analyzing, by an artificial intelligence mapping module (202), the vibration map and identifying patterns to enhance the accuracy and reliability of a system;
identifying and filtering, by a noise correction module (204), unwanted environmental vibrations or noise that interfere with accurate detection of micro-vibrations;
adjusting, by a gain correction module (206), the sensitivity of the one or more sensors to ensure accurate detection of clean vibration signals, optimizing the system performance by accounting for variations in signal strength;
assessing and analyzing, by an environment assessment module (208), the environmental conditions of the medium, and factors comprising temperature, humidity, and external vibrations, to provide context for vibration data;
assessing and analyzing, by a medium assessment module (210), the at least one of pressure, weight distribution, and one or more physiological parameters of the medium;
managing, by a controller (212), the activation, timing, and intensity of the one or more vibration creators (102), ensuring control over the vibration generation process;
supplying, by a power unit (214), power to one or more components of the system (100), ensuring continuous and stable operation;
preparing, by a conditioning unit (216), the detected and captured one or more sensors data for analysis, wherein the analysis comprises amplification, filtering, and other pre-processing of captured one or more sensors data;
transmitting, by a transmitter (218), the one or more sensors data to a remote receiver (222), enabling remote data analysis and real-time monitoring of the medium;
transforming, by a converter (220), input signals into a vectorized format in a windowed fashion, facilitating the capture of time, frequency, and domain-specific signal features, thereby enabling advanced signal processing and analysis; and
collecting and receiving, by the receiver (222), the one or more sensors data transmitted by the transmitter (218), which is further processed or displayed for user monitoring or analysis.

14. A method (400) according to any of claims 10-13, further comprising configuring the artificial intelligence processing unit (108) by:
generating, by a training module (224), at least one training set incorporating clean and standardized signals, and applying one or more of sources, levels, and interactions of the one or more individual signals to produce the clean vibration signals; and
computing, by Fast Fourier Transform (FFT) mapping module (226), at least one Fast Fourier Transform (FFT) of the one or more captured vibration signals.

15. A method (400) according to any of claims 10-14, further comprising monitoring, by the user device (110), the vibrations and analyzing the one or more individual signals, in real-time, provided by the artificial intelligence processing unit (108) and facilitating real-time feedback to a user.
